# EUROPÄISCHE PATENTSCHRIFT

(11) **EP 0 997 157 B1**
(45) Veröffentlichungstag und Bekanntmachung des Hinweises auf die Patenterteilung: **22.02.2006**
(21) Anmeldenummer: 99119385.5
(22) Anmeldetag: 29.09.1999
(51) Int. Cl.: A61M 1/16, A61M 1/34, A61M 1/36

(54) **Blutbehandlungsgerät**
Blood treatment device
Dispositif de traitement du sang

(30) Priorität: 28.10.1998 DE 19849787
(43) Veröffentlichungstag der Anmeldung: 03.05.2000
(73) Patentinhaber: Fresenius Medical Care Deutschland GmbH, 61352 Bad Homburg v.d.H. (DE)
(72) Erfinder: Menninger, Stefan, 97076 Würzburg (DE)
(74) Vertreter: Laufhütte, Dieter

(56) Entgegenhaltungen:
- EP-A- 0 384 155
- DE-A- 3 736 712

## Beschreibung

Die Erfindung betrifft ein Blutbehandlungsgerät mit einer Blutbehandlungskomponente, mit einer Blutleitung zum Transport von Blut zwischen einem Patienten und der Blutbehandlungskomponente, und mit mindestens zwei Kontrolleinheiten zur Überwachung und oder Steuerung des Blutbehandlungsgerätes wobei mindestens zwei Kontrolleinheiten einen Aktionsrechner und einen Hilfsrechner umfassen.

Als Blutbehandlungsgeräte sind Dialysegeräte, Hämofiltrationsgeräte oder auch Blutseparationsgeräte bekannt. Als Blutbehandlungskomponenten werden entsprechend Dialysatoren, Hämofilter, Plasmafilter, Adsorber oder Blutzentrifugen eingesetzt.

Aus der DE 37 36 712 C2 ist ein gattungsgemäßes Blutreinigungsgerät bekannt. Bei dem bekannten Blutreinigungsgerät werden verschiedene Parameter (z.B. Zusammensetzung, Temperatur und Durchsatz der Dialysierflüssigkeit) durch verschiedene Steuereinrichtungen oder Stellglieder (z.B. Pumpen, Heizer, Ventile und Druckregler) gesteuert bzw. geregelt und durch entsprechende Sensoren (z.B. Temperatursensor, Drucksensor, Luftblasensensor) überwacht. Hierbei ist es von entscheidender Bedeutung, die Funktion der einzelnen Kontrolleinheiten sicherzustellen, da ansonsten für den Patienten lebensbedrohliche Situationen entstehen können. Zur Erhöhung der Funktionssicherheit sind gemäß der DE 37 36 712 C2 innerhalb einer Kontrolleinheit redundante Recheneinheiten bestehend jeweils aus einer Steuerprozessoreinheit und einer Monitorprozessoreinheit vorgesehen. Die Kontrolleinheiten untereinander kommunizieren dabei jeweils sternförmig über eine Hauptsteuereinrichtung, wobei die Steuerprozessoreinrichtungen über eine zentrale Hauptsteuerprozessoreinrichtung und die Monitorprozessoreinrichtungen über eine zentrale Hauptmonitorprozessoreinrichtung miteinander kommunizieren.

Ein Nachteil dieser Rechnerorganisation des vorbekannten Blutbehandlungsgeräts besteht allerdings darin, daß ein Ausfall einer zentralen Hauptprozessoreinheit immer noch zu einem Totalausfall des Blutbehandlungsgeräts führen kann.

Aufgabe der Erfindung ist es daher, die Funktionssicherheit des vorbekannten Blutbehandlungsgerätes weiter zu erhöhen.

Diese Aufgabe wird durch ein Blutbehandlungsgerät mit den Merkmalen des Patentanspruchs 1 gelöst.

Aufgrund der direkten Kommunikationsverbindung zwischen dem Aktionsrechner und dem Hilfsrechner innerhalb einer Kontrolleinheit ist eine dezentrale Kommunikation zwischen den einzelnen Kontrolleinheiten möglich, ohne daß der Ausfall einer einzelnen Kontrolleinheit zum Ausfall des gesamten Blutbehandlungsgerätes führt. Je nach Kommunikationsart kann dabei der Aktionsbus und/oder der Hilfsbus beansprucht werden, je nach dem ob es sich bei der sendenden Einheit um einen Aktionsrechner oder einen Hilfsrechner handelt.

Im Gegensatz zu dem aus der DE 37 36 712 C2 bekannten hierarchischen Kommunikationssystem ist dabei eine eigenständige Fehlerbehandlung möglich. Gemäß der DE 37 36 712 C2 wird davon ausgegangen, daß eine Fehlermeldung zunächst zu einer Hauptsteuerprozessoreinrichtung bzw. einer Hauptmonitorprozessoreinrichtung gemeldet werden muß, wobei diese Einrichtungen sodann entscheiden, welche Funktionen die untergeordneten Einheiten aufgrund des Fehlerfalls durchführen müssen. Im Gegensatz dazu ist erfindungsgemäß auf jeder Kontrolleinheit eine Tabelle abgespeichert, die entsprechende Fehlerbehandlungsroutinen für die Kontrolleinheit bereits enthält. Hinsichtlich der Eingänge der Tabelle ist dabei zu unterscheiden zwischen auf der jeweiligen Kontrolleinheit auftretenden Fehlerquellen und Fehlermeldungen, die von außen über den Aktionsbus und/oder den Hilfsbus von der Kontrolleinheit empfangen werden. Fehlerfälle können dabei sowohl von dem Aktionsrechner, als auch von dem Hilfsrechner festgestellt werden. Zweckmäßigerweise ist dabei die Fehlertabelle einer Kontrolleinheit sowohl auf dem Aktionsrechner, als auch auf dem Hilfsrechner gespeichert.

Das erfindungsgemäße Blutbehandlungsgerät umfaßt somit Kontrolleinheiten, die für sich selbständig ablauffähig sind und bei auftretenden Fehlerquellen einen übergeordneten Überwachungsrechner nicht benötigen. Auf diese Weise ist jede Kontrolleinheit eigensicher und kann die Sicherheit der ihr zugewiesenen Aufgabe gewährleisten.

Nach einer bevorzugten Ausführungsform ist vorgesehen, daß der Aktionsrechner bzw. der Hilfsrechner jeweils einer Kontrolleinheit mit Meßgebern und/oder Stellgliedern bzw. mit Meßgebern verbunden ist. Nach einer weiteren bevorzugten Ausführungsform ist vorgesehen, daß der Aktionsrechner und der Hilfsrechner jeweils einer Kontrolleinheit zur Überwachung und Steuerung von für den Patienten lebenswichtigen Komponenten an Meßgebern und Stellgliedern parallel geschaltet sind.

Gemäß einer bevorzugten Ausgestaltung der Erfindung kann die Fehlerbehandlungsroutine in sicherheitsrelevanten Fehlfällen vorsehen, daß sich die jeweilige Kontrolleinheit eigenständig über ihre Stellglieder, die durch den Steuerrechner und/oder den Hilfsrechner angesprochen werden, in einen für die Blutbehandlung sicheren Zustand schalten kann.

Während für lebenswichtige Komponenten ein redundantes Konzept verfolgt wird, kann sich die Steuerung untergeordneter Komponenten auf die Steuerung durch einen selbständig arbeitenden Aktionsrechner oder Hilfsrechner beschränken. Auf diese Weise kann die zur Verfügung stehende Rechenleistung optimal ausgenutzt werden, ohne daß hinsichtlich der Sicherheit am System Abstriche gemacht werden müssen.

Nach einer weiteren bevorzugten Ausführungsform ist vorgesehen, daß bei der Überwachung von lebenswichtigen Komponenten des Blutbehandlungsgerätes ein Alarm ausgelöst wird, wenn der Aktionsrechner und der Hilfsrechner unterschiedliche Ergebnisse liefern. Hierbei erfolgt eine Abschaltung des Blutbehandlungsgerätes in der Weise, daß lebensbedrohliche Situationen für den Patienten auf jeden Fall ausgeschlossen werden können.

Im Falle, daß das Blutbehandlungsgerät ein Dialysegerät ist, werden vorzugsweise eine Bedieneinheit, eine Hydraulikeinheit und eine Funktionseinheit als Kontrolleinheiten vorgesehen. Die Hydraulikeinheit und die Funktionseinheit steuern und überwachen lebenswichtige Komponenten des Blutbehandlungsgerätes, so daß die daran angeschlossenen Sensoren und Aktoren mit Aktionsrechner und Hilfsrechner gesteuert und ausgewertet werden. An den Aktionsrechner der Bedieneinheit sind dabei beispielsweise ein Bildschirm und eine Tastatur und an den Hilfsrechner der Bedieneinheit eine Statusanzeige und eine externe Schnittstelle angeschlossen.

Die Hydraulikeinheit umfaßt zumindest eine Einrichtung zur Dialysierflüssigkeitaufbereitung, Bilanzierung und Ultrafiltration, während die Funktionseinheit zumindest einen optischen Detektor, einen Luftdetektor, einen arteriellen und venösen Drucksensor, eine Blutpumpe, eine Heparin-Pumpe und eine venöse Klemme umfaßt.

Weitere Einzelheiten und Vorteile der Erfindung werden anhand eines in der Zeichnung dargestellten Ausführungsbeispiels in Form eines Dialysegerätes näher erläutert. In dieser zeigt:
- Fig. 1: ein Prinzipschaltbild der Komponenten eines Dialysegerätes und
- Fig. 2: ein Blockschaltbild der Kontrolleinheiten für das Dialysegerät gemäß Fig. 1

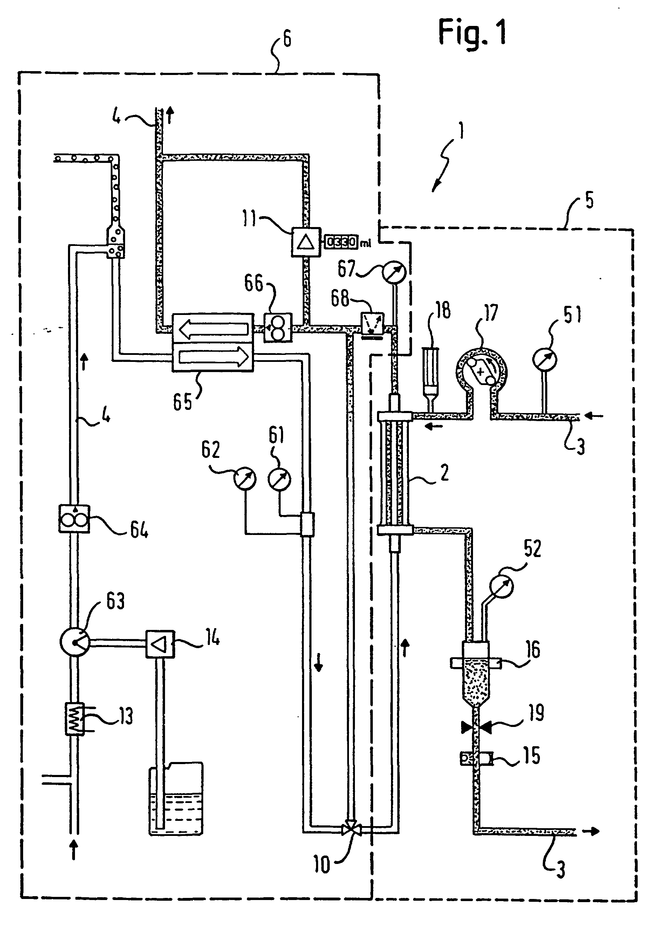

Fig. 1 zeigt ein Prinzipschaltbild der Komponenten eines Dialysegerätes. Wesentlicher Bestandteil des Dialysegerätes 1 ist ein Membranfilter 2, das in eine Blutleitung 3 zum Transport von Blut zwischen einem Patienten und dem Dialysator verschaltet ist. An zwei weitere Stutzen des Membranfilters 2 ist ein Reinigungskreislauf 4 angeschlossen, in dem Dialysierflüssigkeit dem Membranfilter 2 zugeführt und angereichtert mit Schadstoffen aus dem Blut wieder abgeführt wird.

Das Dialysegerät 1 läßt sich folglich in das Blutsystem 5 und in das Hydrauliksystem 6 unterteilen, wobei das Blutsystem 5 von der Funktionseinheit 21 und das Hydrauliksystem 6 von der Hydraulikeinheit 22 gesteuert werden.

Seinem wesentlichen Aufbau nach umfaßt das Blutsystem 5 eine Blutpumpe 17, eine Heparinpumpe 18, einen Luftdetektor 16, eine venöse Klemme 19 sowie einen optischen Detektor 15 sowie einen arteriellen und einen venösen Drucksensor 51, 52.

Wesentliche Komponenten des Hydrauliksystems 6 sind demgegenüber die UF-Pumpe 11, das Bypass-Ventil 10, die Heizung 13, das Mischsystem 14, die Entgasungspumpe 64, die Bilanziereinheit 65, die Dialysierflüssigkeitspumpe 66 der Transmembranderuckmonitor 67 sowie der Blutleckdetektor 68. Vor dem Mischpunkt 63 wird das zugeführte Wasser durch die Heizung 13 erwärmt. Die Konzentrationspumpe 14 mischt die Dialysierflüssigkeitskonzentrate mit Wasser in einem vom Bediener vorgegebenen Verhältnis. Die Entgasungspumpe 64 wird weiterhin so gesteuert, daß in dem Kreislauf 4 nur entgaste Flüssigkeiten zirkulieren. In der Bilanzkammer 65 wird der Dialysatfluß zum und vom Dialysator 2 symmetriert, und es wird eine genaue Menge an Ultrafiltrat entfernt, indem durch Steuerung der UF-Pumpe 11 die gewünschte Menge abgezogen wird. Die gewünschte Strömungsrate im Kreislauf 4 ergibt sich durch die Dialysierflüssigkeitspumpe 66 und einem darauf abgestimmten Wechselspiel der Zu- und Abflußventile der nicht näher gezeigten, zwei parallel geschalteten Bilanzkammern der Bilanziereinheit 65. Mit dem UF-Drucksensor 67 wird dabei der Transmembrandruck überwacht. Mit dem Blutleckdetektor 68 läßt sich schließlich ein Blutleck im Dialysator 2 feststellen.

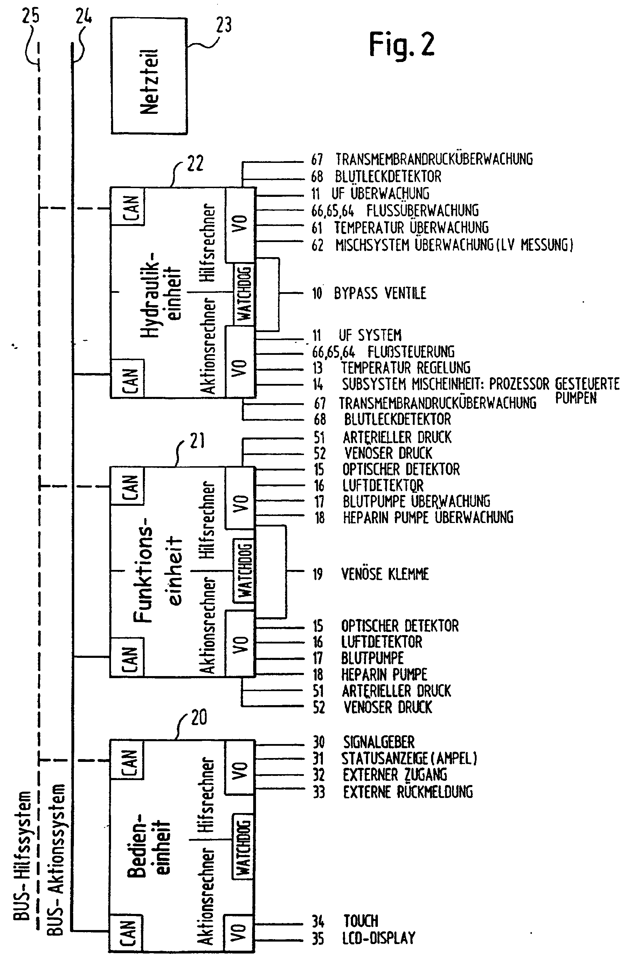

Fig. 2 zeigt das Blockschaltbild der Kontrolleinheiten für das Dialysegerät gemäß Fig. 1. Als Kontrolleinheiten sind eine Bedieneinheit 20, eine Funktionseinheit 21 sowie eine Hydraulikeinheit 22 vorgesehen. Die einzelnen Kontrolleinheiten bestehen dabei jeweils aus einem Aktionsrechner und einem Hilfsrechner.

Sämtliche Kontrolleinheiten werden über ein Netzteil 23 mit Spannung versorgt, wobei das Netzteil 23 seinerseits redundante Komponenten aufweist, um einen Ausfall des gesamten Dialysegerätes beim Ausfall eines Netzteils zu verhindern.

Die Aktionsrechner jeder Kontrolleinheit sind über einen Aktionsbus 24 und die Hilfsrechner über einen Hilfsbus 25 miteinander verbunden. Zur Kommunikation zwischen Aktionsrechnern verschiedener Kontrolleinheiten wird seitens des sendenden Aktionsrechners eine adressierte Nachricht an den Aktionsbus gelegt, die aufgrund der Adressierung zu dem jeweiligen als Empfänger dienenden Aktionsrechner gelangt. Weiterhin ist eine Kommunikation innerhalb jeder Kontrolleinheit zwischen einem Aktionsrechner und einem Hilfsrechner möglich, so daß Nachrichten auch von einem Aktionsrechner einer Kontrolleinheit zu einem Hilfsrechner einer anderen Kontrolleinheit bzw. umgekehrt von einem Hilfsrechner einer Kontrolleinheit zu einem Aktionsrechner einer anderen Kontrolleinheit gesendet werden können. Neben dem aktiven Senden von Nachrichten von einer Kontrolleinheit zu einer anderen Kontrolleinheit ist es auch möglich, daß bestimmte Informationen von anderen Kontrolleinheiten angefordert werden. Der Erhalt der angeforderten Informationen wird in üblicher Weise über Zeitablauf gesteuert. Bleiben die Informationen aus, so müssen von der jeweiligen Kontrolleinheit weitere Konsequenzen getroffen werden, wie z.B. Abbruch der Kommunikation mit der jeweiligen Kontrolleinheit und Mitteilung an eine andere Kontrolleinheit. Der Verlust einer Kontrolleinheit führt dabei nicht zwangsweise zum Ausfall des gesamten Dialysegerätes, da die anderen Kontrolleinheiten weiterhin miteinander kommunizieren können.

Die Meß- und Stellglieder der Hydraulik- und Funktionseinheit können dabei parallel an den jeweiligen Aktions- und Hilfsrechner angeschlossen sein oder redundant ausgebildet sein.

An den Hilfsrechner der Bedieneinheit ist dabei ein Signalgeber 30, eine Statusanzeige 31, ein externer Zugang 32 sowie eine externe Rückmeldung 33 angeschlossen. An den Aktionsrechner der Bedieneinheit 20 ist dagegen ein Tastenfeld 34 sowie ein LCD-Display 35 angeschlossen.

Die erfindungsgemäße Fehlerbehandlung wird an dem folgenden Beispiel erläutert.

Die Einstellungen für das Mischsystem 14 werden von dem Bediener an der Bedieneinheit 20 eingestellt. Die entsprechenden Eingaben auf dem Tastenfeld 34 werden dabei vom Aktionsrechner der Bedieneinheit 20 ausgewertet. Eine Rückmeldung an den Bediener geschieht über das LCD-Display 35. Die neuen Vorgabe werte werden über den Aktionsbus 24 allen anderen Aktionsrechnern der anderen Kontrolleinheiten mitgeteilt.

Der Aktionsrechner der Hydraulikeinheit 22 rechnet aus der Vorgabe neue Einstellwerte für das aktive Mischsystem 14, wobei die Werte der Mischeinheit 14 übergeben werden.

Der Hilfsrechner der Hydraulikeinheit 22 führt die gleichen Rechenoperationen wie der entsprechende Aktionsrechner aus, wobei zusätzliche Toleranzschwellen vorgesehen sind, bei deren Überschreitung aufgrund einer Abweichung zwischen dem Aktionsrechner und dem Hilfsrechner eine entsprechende Fehlermeldung ausgegeben wird. Des weiteren kontrolliert der Hilfsrechner über den Leitfähigkeitssensor 62 die Zusammensetzung der Dialysierflüssigkeit.

Wird ein Fehler im Mischsystem vom Hilfsrechner der Hydraulikeinheit 22 erkannt, beispielsweise weil die Zusammensetzung des Dialysats aufgrund der Messung der LF-Zelle nicht in den gewünschten Grenzen liegt, so steuert die Hydraulik-Einheit sofort die Bypass-Ventile 10 derart an, daß der Dialysierflüssigkeitsfluß den Dialysator umgeht und der Patient geschützt wird. Über den Hilfsbus 25 wird der aufgrund der LF-Überwachung ausgelöste Alarm allen anderen angeschlossenen Hilfsrechnern mitgeteilt. Jeder Hilfsrechner leitet dabei selber die nötigen Maßnahmen ein. Seitens der Bedieneinheit 20 schaltet der Hilfsrechner die Statusanzeige 31 auf rot und gibt über den Signalgeber 30 einen Alarmton aus. Der Alarm wird weiterhin dem Aktionsrechner der Bedieneinheit 20 mitgeteilt. Der Aktionsrechner der Bedieneinheit 20 zeigt dem Bediener wiederum über das LCD-Display 35 eine Beschreibung des Alarms an. Die Aktions- und Hilfsrechner der Funktionseinheit 21 entscheiden nun selbst, welche Konsequenzen aus dem Alarmzustand zu ziehen sind. Im vorliegenden Fall wird die Zirkulation des Blutes im extrakorporalen Kreislauf eine zeitlang aufrechterhalten. Wenn nach einer gewissen Zeitspanne (z. B. 10 Minuten) keine Eingaben an der Bedieneinheit vorgenommen werden, werden die Blutpumpe angehalten und die venöse Klemme geschlossen.

## Patentansprüche

1. Blutbehandlungsgerät
mit einer Blutbehandlungskomponente,
mit einer Blutleitung (3) zum Transport von Blut zwischen einem Patienten und der Blutbehandlungskomponente, und
mit mindestens zwei Kontrolleinheiten (20, 21, 22) zur Überwachung und/oder Steuerung des Blutbehandlungsgerätes (1), wobei mindestens zwei Kontrolleinheiten einen Aktionsrechner und einen Hilfsrechner umfassen,
**dadurch gekennzeichnet,**
**daß** die Aktionsrechner miteinander über einen Aktionsbus (24) und die Hilfsrechner miteinander über einen Hilfsbus (25) verbunden sind,
**daß** innerhalb der mindestens zwei Kontrolleinheiten (20, 21, 22) eine Kommunikation zwischen dem Aktionsrechner und dem Hilfsrechner vorgesehen ist und
**daß** in jeder der mindestens zwei Kontrolleinheiten (20, 21, 22) auf dem Aktionsrechner und/oder auf dem Hilfsrechner eine Tabelle abgespeichert ist, in der auf der jeweiligen Kontrolleinheit und/oder durch die Buskommunikation auftretende Fehlerfälle und/oder von der jeweiligen Kontrolleinheit empfangene Fehlermeldungen einer Fehlerbehandlungsroutine zugeordnet sind,
wobei der Aktionsrechner und/oder der Hilfsrechner jeder der mindestens zwei Kontrolleinheiten (20, 21, 22) derart ausgestaltet ist/sind, daß er/sie eine Fehlermeldung auf dem zugehörigen Bus absetzt/absetzen, sobald ein Fehlerfall auftritt und/oder eine Fehlermeldung empfangen wird und die dem Fehlerfall und/oder der Fehlermeldung zugeordnete Fehlerbehandlungsroutine dies vorsieht.

2. Blutbehandlungsgerät nach Anspruch 1, **dadurch gekennzeichnet, daß** die Aktionsrechner und/oder Hilfsrechner der mindestens zwei Kontrolleinheiten (20, 21, 22) mit Meßgebern und/oder Stellgliedern verbunden sind.

3. Blutbehandlungsgerät nach Anspruch 2, **dadurch gekennzeichnet, daß** der Aktionsrechner und der Hilfsrechner jeweils einer Kontrolleinheit (20, 21, 22) zur Überwachung und/oder Steuerung von für den Patienten lebenswichtigen Komponenten parallel an den Meßgebern und/oder Stellgliedern geschaltet sind.

4. Blutbehandlungsgerät nach Anspruch 3, **dadurch gekennzeichnet, daß** sie derart ausgebildet ist, daß ein Alarm ausgelöst werden kann, wenn der Aktionsrechner und der Hilfsrechner unterschiedliche Ergebnisse liefern.

5. Blutbehandlungsgerät nach einem der Ansprüche 1-4, **dadurch gekennzeichnet, daß** die Fehlerbehandlungsroutine derart ausgebildet ist, daß die jeweilige Kontrolleinheit (20, 21, 22) bei Auftreten bestimmter Fehler eigenständig über ihr zugeordnete Stellglieder in einen für die Blutbehandlung sicheren Zustand schalten kann.

6. Blutbehandlungsgerät nach einem der Ansprüche 1-5, **dadurch gekennzeichnet, daß** das Blutbehandlungsgerät ein Dialysegerät (1) ist und daß als Kontrolleinheiten eine Bedieneinheit (20), eine Hydraulikeinheit (21) und eine Funktionseinheit (22) vorgesehen sind.

7. Blutbehandlungsgerät nach Anspruch 6, **dadurch gekennzeichnet, daß** an den Aktionsrechner der Bedieneinheit ein Bildschirm (35) und eine Tastatur (34) und an den Hilfsrechner der Bedieneinheit eine Statusanzeige (31) und eine externe Schnittstelle (32, 33) angeschlossen sind.

8. Blutbehandlungsgerät nach Anspruch 6 oder 7, **dadurch gekennzeichnet, daß** die Hydraulikeinheit (22) eine Dialysierflüssigkeitsaufbereitung, eine Bilanziereinheit (65) und eine Ultrafiltrationsvorrichtung umfaßt.

9. Blutreinigungsgerät nach einem der Ansprüche 6 bis 8, **dadurch gekennzeichnet, daß** an die Funktionseinheit ein optischer Detektor (15), ein arterieller und ein venöser Drucksensor (51, 52), ein Lufdetektor (16), eine Blutpumpe (17), eine Heparin-Pumpe (18) und eine venöse Klemme (19) angeschlossen sind.

## Claims

1. A blood treatment apparatus comprising
a blood treatment component,
a blood conduit (3) for transporting blood between a patient and the blood treatment component, and
at least two controlling units (20, 21, 22) for monitoring and/or controlling the blood treatment apparatus (1), wherein at least two controlling units include an action computer and an auxiliary computer,
**characterised in that**
the action computers are connected together by way of an action bus (24) and the auxiliary computers are connected together by way of an auxiliary bus (25),
a communication is provided between the action computer and the auxiliary computer within the at least two controlling units (20, 21, 22), and
stored in each of the at least two controlling units (20, 21, 22) on the action computer and/or on the auxiliary computer is a table in which error situations occurring on the respective controlling unit and/or due to the bus communication and/or error messages received by the respective controlling unit are associated with an error processing routine,
wherein the action computer and/or the auxiliary computer of each of the at least two controlling units (20, 21, 22) is/are so designed that it/they puts/put an error message on the associated bus as soon as an error situation occurs and/or an error message is received and the error processing routine associated with the error situation and/or the error message provides same.

2. A blood treatment apparatus according to claim 1 **characterised in that** the action computers and/or auxiliary computers of the at least two controlling units (20, 21, 22) are connected to measuring sensors and/or setting members.

3. A blood treatment apparatus according to claim 2 **characterised in that** the action computer and the auxiliary computer of a respective controlling unit (20, 21) for monitoring and/or controlling components which are vital for the patient are connected in parallel to the measuring sensors and/or setting members.

4. A blood treatment apparatus according to claim 3 **characterised in that** it is so designed that an alarm can be triggered if the action computer and the auxiliary computer produce different results.

5. A blood treatment apparatus according to one of claims 1 to 4 **characterised in that** the error processing routine is such that the respective controlling unit (20, 21, 22) can switch into a condition safe for blood processing upon the occurrence of given errors independently by way of setting members associated therewith.

6. A blood treatment apparatus according to one of claims 1 to 5 **characterised in that** the blood treatment apparatus is a dialysis apparatus (1) and that provided as the controlling units are an operating unit (20), a hydraulic unit (21) and a function unit (22).

7. A blood treatment apparatus according to claim 6 **characterised in that** a display screen (35) and a keyboard (34) are connected to the action computer of the operating unit and a status display (31) and an external interface (32, 33) are connected to the auxiliary computer of the operating unit.

8. A blood treatment apparatus according to claim 6 or claim 7 **characterised in that** the hydraulic unit (22) includes dialysis fluid processing, a balancing unit (65) and an ultrafiltration device.

9. A blood treatment apparatus according to one of claims 6 to 8 **characterised in that** an optical detector (15), an arterial and a venous pressure sensor (51, 52), an air detector (16), a blood pump (17), a heparin pump (18) and a venous clamp (19) are connected to the function unit.

## Revendications

1. Appareil de traitement du sang
avec des composants du traitement du sang ,
avec une conduite de sang (3) pour le transport du sang entre un patient et les composants de traitement du sang, et
avec au moins deux unités de contrôle (20,21,22) pour la surveillance et/ou la commande de l'appareil de traitement du sang (1) où au moins deux unités de contrôle comprennent un calculateur actif et un calculateur auxiliaire,
**caractérisé en ce que**
les calculateurs actifs sont reliés ensemble au moyen d'un bus actif (24) et les calculs auxiliaires au moyen d'un bus auxiliaire (25),
**en ce qu'**à l'intérieur des au moins deux unités de contrôle (20,21,22) est prévue une communication entre le calculateur actif et le calculateur auxiliaire et
**en ce que** dans chacune des au moins deux unités de contrôle (20,21,22) sur le calculateur actif et/ou le calculateur auxiliaire est mémorisé un tableau dans lequel, pour chaque cas d'erreur et/ou chaque avis d'erreur se présentant à chaque unité de contrôle et/ou au bus de communication reçu par une unité de contrôle sont coordonnées une routine de traitement de l'erreur ,
ce par quoi le calculateur actif et/ou le calculateur auxiliaire de chacune des au moins deux unités de contrôle (20,21,22) sont configurés de façon à appliquer un avis d'erreur au bus correspondant des qu'un cas d'erreur se présente et/ou qu'un avis d'erreur a été reçu et celui-ci prévoit la routine de traitement de l'erreur correspondant au cas d'erreur et/ou à l'avis d'erreur.

2. Appareil de traitement du sang selon la revendication 1, **caractérisé en ce que** le calculateur actif et/ou le calculateur auxiliaire des au moins deux unités de contrôle (20,21,22) sont reliés à des indicateurs de mesure et/ou des organes de réglage.

3. Appareil de traitement du sang selon la revendication 1, **caractérisé en ce que** le calculateur actif et le calculateur auxiliaire sont reliés à une unité de contrôle (20,21,22) pour la surveillance et/ou la commande des composants vitaux pour le patient en parallèle avec les indicateurs de mesure et/ou les organes de réglage.

4. Appareil de traitement du sang selon la revendication 3, **caractérisé en ce qu'**il est configuré de façon qu'une alarme soit déclenchée quand le calculateur actif et le calculateur auxiliaire donnent des résultats différents.

5. Appareil de traitement du sang selon l'une quelconque des revendications 1-4, **caractérisé en ce que** la routine de traitement de l'erreur est configurée de façon que l'unité de contrôle correspondante (20,21,22), lorsque se présentent des erreurs déterminées, puisse, par l'intermédiaire des organes de réglage qui lui sont affectés, passer à un état de sécurité pour le traitement du sang.

6. Appareil de traitement du sang selon l'une quelconque des revendications 1-5, **caractérisé en ce que** l'appareil de traitement du sang est un appareil de dialyse (1) et **en ce que** sont prévues, en tant qu'unités de contrôle, une unité de manoeuvre (20), une unité hydraulique (21) et un ensemble opérationnel (22).

7. Appareil de traitement du sang selon la revendication 6, **caractérisé en ce qu'**au calculateur actif de l'unité de manoeuvre sont raccordés un écran (35) et un clavier (34) et au calculateur auxiliaire de l'unité de manoeuvre sont raccordés un indicateur d'état (31) et une interface externe (32,33).

8. Appareil de traitement du sang selon la revendication 6 ou 7, **caractérisé en ce que** l'unité hydraulique (22) comporte une préparation du liquide de dialyse, une unité d'équilibrage (65) et un dispositif d'ultrafiltration.

9. Appareil de purification du sang selon l'une quelconque des revendications 6 à 8, **caractérisé en ce qu'**à l'ensemble fonctionnel sont raccordés un détecteur optique (15), un capteur de pression artérielle et veineuse (51,52), un détecteur d'air (16), une pompe à sang (17), une pompe à héparine (18) et une pince veineuse (19).
